# FASCICULE DE BREVET EUROPEEN

(11) **EP 3 297 538 B1**
(45) Date de publication et mention de la délivrance du brevet: **20.03.2019**
(21) Numéro de dépôt: 16726136.1
(22) Date de dépôt: 09.05.2016
(51) Int. Cl.: A61B 6/10, A61B 6/00

(54) **DISPOSITIF DE SIGNALISATION DU STATUT D'UN APPAREIL A EMISSION RADIOELECTRIQUE EN EXTERIEUR, ET NOTAMMENT D'UN APPAREIL MUNI D'UN TUBE A RAYON X**
VORRICHTUNG ZUR SIGNALISIERUNG DES STATUS EINER FUNKÜBERTRAGUNGSVORRICHTUNG IN DIE UMGEBUNG, INSBESONDERE EINER VORRICHTUNG MIT EINER RÖNTGENRÖHRE
DEVICE FOR SIGNALLING INTO THE ENVIRONMENT THE STATUS OF A RADIO TRANSMISSION APPARATUS, AND IN PARTICULAR OF AN APPARATUS PROVIDED WITH AN X-RAY TUBE

(30) Priorité: 19.05.2015 FR 1554474
(43) Date de publication de la demande: 28.03.2018
(73) Titulaire: Biomediqa, 59650 Villeneuve d'Ascq (FR)
(72) Inventeur: MAALOUL, Fouad, 59700 Marcq en Baroeul (FR); GUERIN, Laura, 59000 Lille (FR)
(74) Mandataire: Schwalek, Valérie
(86) Numéro de dépôt international: PCT/FR2016/051081
(87) Numéro de publication internationale: WO 2016/185112

(56) Documents cités:
- EP-A1- 2 329 772
- CN-U- 202 568 292
- JP-A- 2014 138 667

## Description

### DOMAINE TECHNIQUE

La présente invention concerne un dispositif de signalisation du statut d'un appareil à émission radioélectrique en champs ouvert, et notamment d'un appareil muni d'un tube à rayon X. L'invention sera particulièrement destinée à venir équiper des appareils de radiologie mobiles dans les hôpitaux ou cliniques médicales.

Toutefois, cette utilisation n'est pas limitative, le dispositif pourra notamment être associé de manière plus large à tout type d'appareils à émission radioélectrique, à poste fixe ou mobile, dont les émissions peuvent engendrer des problèmes de sécurité et également sur des appareils équipant des laboratoires de recherche ou encore des installations industrielles.

### ART ANTERIEUR

Les hôpitaux disposent en général de salles dédiées à l'utilisation d'appareils à émission radioélectriques (repris dans le reste de la présente demande sous l'expression « appareil à émission »), il s'agit notamment de salles de radiologie conventionnelle ou encore de salles pour scanner ou pour gamma-caméra.

En général ces salles sont équipées d'appareils à émission fixes, et comprennent des moyens de protection contre les rayonnements. Parmi ces moyens de protection, il est généralement prévu une signalétique passive avec des inscriptions particulières informant de la présence possible de rayonnements ionisants. En complément, ces salles comportent également des panneaux lumineux indiquant si les appareils à émission sont activés ou non. Ces salles, dédiées à la réception d'appareils à émission, nécessitent des travaux d'installation spécifiques et notamment de câblage entre les panneaux lumineux et les appareils à émission.

Cette solution, bien que couteuse et nécessitant une fermeture temporaire des salles à équiper, est relativement satisfaisante. Elle n'est toutefois pas adapté aux appareils à émission mobile, il existe en effet un certain nombre d'appareils à émission facilement déplaçables d'une salle à l'autre, et couramment utilisés dans des salles non dédiées et par exemple dans les blocs opératoires.

Parmi ces appareils à émission, on retrouve par exemple des appareils munis d'un tube à rayon X ou amplificateur de brillance, ces derniers étant déplacés selon les besoins d'un bloc opératoire à un autre.

On comprend bien que l'usage d'appareils à émission particulièrement dans des salles non dédiées est problématique pour la sécurité du personnel médical et des patients puisqu'aucun moyen de signalisation n'informe des risques de présence de rayonnements.

Il est bien entendu possible d'indiquer le risque encouru dans toutes les salles susceptibles de recevoir des appareils à émission, cela étant, une signalétique constante dans toutes les salles rendrait soit les déplacements difficiles dans l'établissement soit diminuerait l'impact de l'avertissement sur les utilisateurs et ce d'autant que ces salles ne sont, en définitive, soumises qu'épisodiquement aux rayonnements.

Le document CN202568292U divulgue un dispositif de sécurité pour une salle dédiée à l'utilisation d'appareils à émission radioélectrique qui coupe l'alimentation du dispositif a émission lorsque la porte d'accès à la salle est ouverte.

### OBJET DE L'INVENTION

Un premier but de la présente invention est de résoudre tout ou partie des problèmes techniques liés à l'art antérieur précité.

Un autre but de la présente invention est de proposer un dispositif de signalisation permettant de signaler une émission de rayonnement en présence d'un appareil à émission dans une salle sans nécessité d'aménagement particulier de la salle.

Un autre but de la présente invention est de proposer un dispositif de signalisation pouvant être adapté de manière rapide et fiable sur tout type d'appareil à émission à poste fixe ou mobile.

Un autre but de la présente invention est de proposer un dispositif de signalisation dans lequel la signalétique peut être multiple et facilement déplaçable pour être visible de tous les points d'accès à la salle.

Un autre but de la présente invention est de proposer un dispositif de signalisation permettant de stopper l'appareil à émission en cas de risque d'exposition, notamment dans des installations industrielles.

### RESUME DE L'INVENTION

La présente invention concerne un dispositif de signalisation du statut d'un appareil à émission radioélectrique en extérieur, et notamment d'un appareil muni d'un tube à rayon X, et tel qu'il comprend, selon l'invention :
- une première partie, apte à être connectée au niveau du câble d'alimentation dudit appareil à émission, et comportant des moyens de mesure de la demande en courant dudit appareil à émission, des moyens de traitement permettant de déterminer le statut, hors tension, veille ou utilisation, dudit appareil à émission en fonction de la mesure du courant et des moyens de transmission du statut détecté vers une seconde partie,
- une seconde partie comportant des moyens de signalisation du statut de l'appareil à émission détecté par ladite première partie.

### DEFINITION

Le terme «appareil à émission radioélectrique en extérieur» définit, au sens de la présente invention, tout appareil générant des émissions radioélectriques hors de sa structure.

### BREVE DESCRIPTION DES FIGURES

La présente invention sera mieux comprise à la lecture d'un exemple détaillé de réalisation en référence aux figures annexées, fournies à titre d'exemple non limitatif, parmi lesquels :
- la figure 1 représente un exemple de réalisation schématique d'un dispositif de signalisation conforme à l'invention,
- la figure 2 représente un exemple de réalisation, en vue de perspective d'un dispositif de signalisation placé sur un appareil à émission disposé dans une salle,
- la figure 3 représente un graphique des besoins en courant d'un appareil à émission en fonction de son statut,
- la figure 4 représente un exemple d'ensemble comportant deux dispositifs de signalisation avec une unité centrale.

### DESCRIPTION DETAILLEE DE L'INVENTION

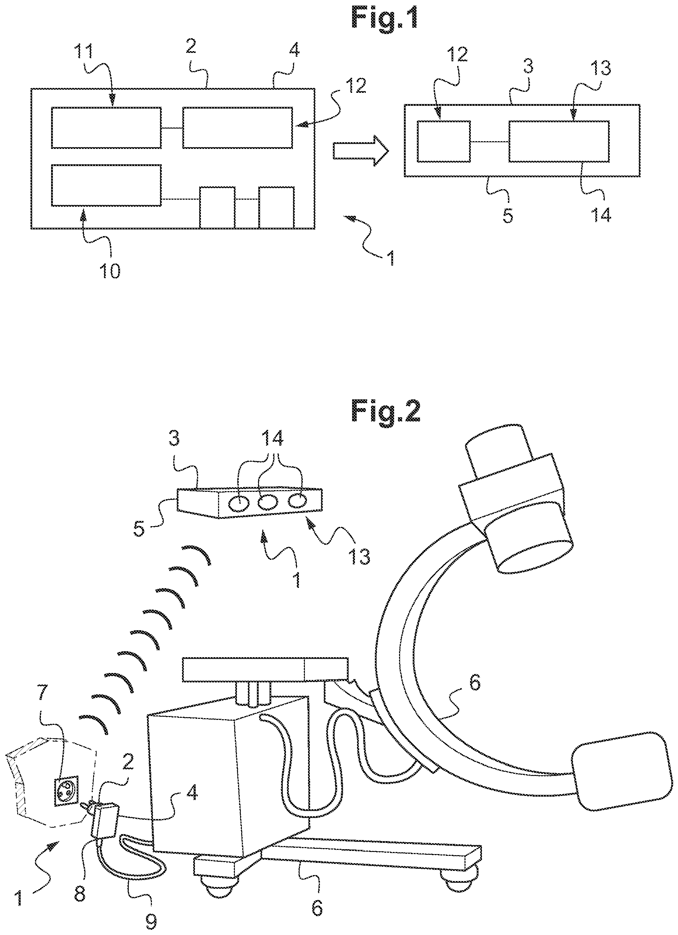

La présente invention vise à protéger un dispositif de signalisation du statut d'un appareil à émission radioélectrique en extérieur. En se reportant principalement à la figure 1 on voit représenté, sous forme schématique un exemple de réalisation de ce dispositif de signalisation 1.

Ce dispositif de signalisation 1 comprend deux parties, à savoir une première partie 2 destinée à être connectée au niveau du câble d'alimentation d'un appareil à émission et une seconde partie 3 destinée à être positionnée avantageusement devant un accès de la salle.

De préférence comme représenté à la figure 1 ces deux parties 2 et 3 sont disposées dans des boitiers séparés respectivement 4 et 5, toutefois dans un autre mode de réalisation envisageable, l'ensemble des éléments du dispositif 1 seront réunis dans un boitier unique.

En se reportant à la figure 2 représentant le dispositif 1 en situation de fonctionnement sur un appareil 6, on voit que la première partie 2 vient se brancher entre la source d'alimentation 7 de l'appareil et la fiche 8 du câble d'alimentation 9.

La première partie 2 comprend des moyens de mesure 10 de la demande en courant dudit appareil à émission 6. Ces moyens de mesure 10 sont de préférence réalisé avec une pince ampéremétrique permettant de mesurer l'intensité du courant requis par l'appareil à émission. Cela étant, d'autres dispositifs de mesure du courant, connus de l'homme du métier, sont également envisageables à la place de ladite pince ampéremétrique.

La première partie 2 comporte en outre des moyens de traitement 11 permettant de déterminer le statut, hors tension, veille ou utilisation, dudit appareil à émission en fonction de la mesure du courant effectuée par les moyens de mesure 10.

De manière avantageuse, les moyens de traitement 11 permettent un autoapprentissage des seuils bas et haut lors respectivement de la première mise en veille et mise en fonctionnement de l'appareil à émission 6.

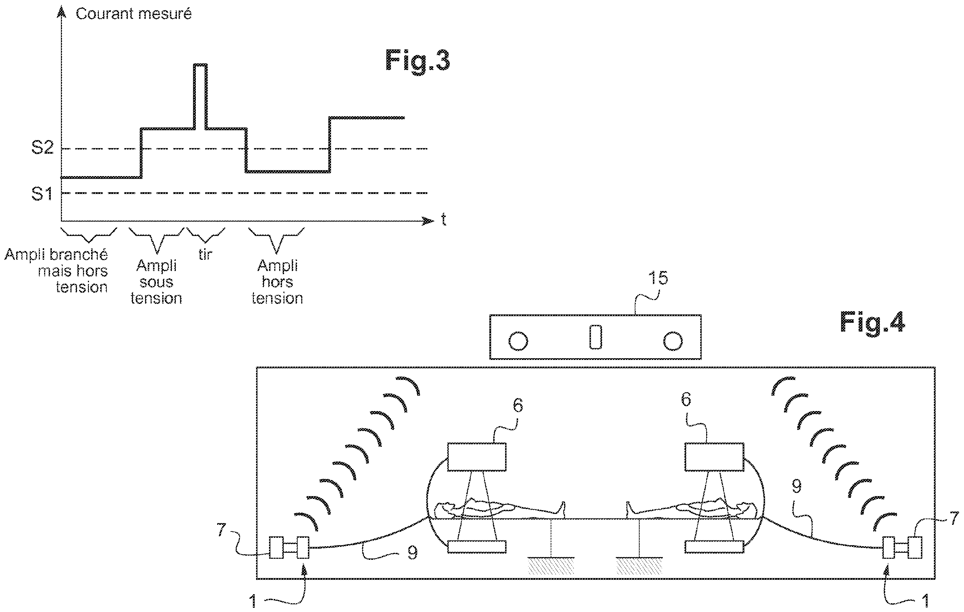

En se reportant à la figure 3 on voit représenté un graphique des besoins en courant d'un appareil à émission 6, par exemple un amplificateur de brillance, en fonction de son statut. Pour cet amplificateur de brillance on retrouve un premier niveau de besoin lorsque l'appareil 6 est en veille, puis un second niveau correspondant au même dispositif en utilisation.

Il est important de noter que dans la présente demande on entend par veille, l'état dans lequel est l'appareil 6 lorsqu'il est branché mais n'émet pas de rayonnement. Dans cet état de veille l'appareil 6 peut toutefois exécuter des commandes ou encore des calculs. On entend en outre par l'expression « en utilisation » l'état dans lequel l'appareil 6 émet des rayonnements.

Les moyens de traitement 11 permettent de créer automatiquement un premier seuil bas S1 en dessous de la quantité de courant requis par la veille, ce seuil S1 permettant de repérer que l'appareil 6 est susceptible d'émettre un rayonnement et un second seuil haut S2 représentant une valeur de courant supérieure à S1 et inférieure à la demande minimale en courant de l'appareil en utilisation. Avantageusement la valeur du seuil S2 est proportionnelle à celle de S1. Ce second seuil S2 lorsqu'il est franchi vers le haut permet de repérer que l'appareil 6 est en émission ou en préparation d'émission.

Dans le mode de réalisation préféré, les moyens de traitement comportent donc au moins deux seuils dont un premier seuil bas S1 est inférieur à la demande minimale en courant lorsque l'appareil est en veille et un second seuil S2 haut est inférieur à la demande minimale en courant de l'appareil en utilisation. Toutefois dans une version simplifiée, on prévoit que les moyens de traitement 11 ne comportent qu'un seul seuil correspondant à S2.

A l'inverse dans une autre version on prévoit en outre un troisième seuil S3 correspondant à une valeur intermédiaire entre S1 et S2 permettant de distinguer entre la demande en courant préparatoire à l'émission et la demande en courant lors de l'émission. Ce seuil S3 permet de signaler un statut supplémentaire de l'appareil à savoir une préparation d'émission.

Dans un autre mode de réalisation, les moyens de traitement 11 ne comportent pas de moyens d'autoapprentissage mais comportent une mémoire comportant des seuils haut et bas fixes préenregistrés en fonction des appareils à émission auquel le dispositif de signalisation 1 correspond.

La première partie 2 comporte en outre des moyens de transmission 12 du statut détecté de l'appareil 6 vers la seconde partie 3.

Dans le mode de réalisation illustré, les première et seconde parties 2 et 3 sont logées dans des boitiers indépendants 4 et 5, et lesdits moyens de transmission 12 comportant une liaison sans fil.

Cette caractéristique est particulièrement intéressante puisqu'elle permet de placer la seconde partie 2 comportant des moyens de signalisation 13 dans un endroit visible depuis les accès de la salle sans contrainte de câblage. A ce sujet, le boîtier 5 permet de loger une batterie permettant d'assurer l'autonomie de la seconde partie 2.

De manière connue en soit les moyens de transmission 12 sans fil sont réalisés à partir d'un émetteur récepteur radioélectrique et par exemple de type Bluetooth, ou de type NFC. De manière optionnelle, la liaison entre les deux parties 2 et 3 peut être codée de manière à éviter tout risque d'interférence et/ou d'erreur d'appairage entre par exemple différents dispositifs de signalisation 1 disposés dans des salles géographiquement proches.

Les moyens de signalisation 13 sont de préférence lumineux et/ou sonores. Selon un premier mode de réalisation avantageux, on prévoit au niveau de la seconde partie 3 au moins une lampe 14 permettant de réaliser un code de signalisation couleur. Selon un autre mode de réalisation, la seconde partie 3 comprend un afficheur permettant d'afficher des textes notamment correspondant au statut de l'appareil 6 identifié et transmis par la première partie 2.

En se reportant cette fois à la figure 4, on voit représenté une salle équipée de plusieurs appareils à émission 6, chacun relié à un dispositif de signalisation 1. Dans cette configuration, on prévoit une unité centrale d'affichage 15 permettant d'afficher un seul signal ou message en fonction du statut de chaque appareil à émission 6.

Ainsi, l'unité centrale 15 affiche le signal correspondant au degré de statut le plus élevé atteint par un des appareils à émission relevé dans la salle, le statut le plus élevé étant bien entendu un appareil 6 en cours d'utilisation.

De préférence, l'unité centrale 15 est réalisée avec une seconde partie 3 appairée avec l'ensemble des premières parties 2 des dispositifs de signalisation 1 présents dans la salle

Dans une variante de réalisation, l'unité centrale 15 peut également afficher séparément le statut de chaque dispositif de signalisation 1 auxquels il est relié avec une identification du code ou du nom de chaque dispositif 1.

La présente invention vise ainsi également à protéger un ensemble comportant une unité centrale permettant de recevoir le signal émis par au moins une première partie 2 du dispositif de signalisation 1 et permettant de collecter les données de statut relatives à plusieurs dispositifs de signalisation.

Dans une variante de réalisation, on prévoit que le dispositif 1 comprend, au niveau des moyens de traitement 11, des moyens de suivi du nombre d'heures d'utilisation des appareils à émission soit appareil par appareil soit zone d'appareils par zone d'appareils sur une durée déterminée. L'historique d'utilisation de chaque appareil est pour ce faire transmis à une unité centrale qui collecte l'ensemble des données relatives aux différents dispositifs concernés. Ces données sont ensuite traitées pour réaliser des études de risques et déterminer des zones à risque variable ou encore pour programmer des opérations de maintenance des appareils à émission.

Bien entendu, d'autres caractéristiques de l'invention auraient également pu être envisagées sans pour autant sortir du cadre de l'invention définie par les revendications ci-après.

A titre d'exemple, le dispositif de signalisation 1 comprend dans une variante de réalisation la première partie comportent des moyens de coupure de l'alimentation de l'appareil à émission couplés à des moyens de détection de présence dans la zone de champs ouvert de l'appareil à émission. Ces moyens de détection pourront être notamment des détecteurs de type ponctuel notamment au niveau des passages de portes ou encore de détecteurs volumétriques.

## Revendications

1. Dispositif de signalisation du statut d'un appareil à émission radioélectrique en extérieur, et notamment d'un appareil muni d'un tube à rayon X, **caractérisé en ce qu'**il comprend :
- une première partie (2), apte à être connectée au niveau du câble d'alimentation (9) dudit appareil à émission, et comportant des moyens de mesure (10) de la demande en courant dudit appareil à émission, des moyens de traitement (11) permettant de déterminer le statut, hors tension, veille ou utilisation, dudit appareil à émission en fonction de la mesure du courant et des moyens de transmission (12) du statut détecté vers une seconde partie (3),
- une seconde partie (3) comportant des moyens de signalisation (13) du statut de l'appareil à émission détecté par ladite première partie.

2. Dispositif de signalisation selon la revendication 1 dans lequel la première (2) et la seconde (3) partie sont logés dans des boitiers indépendants (4,5), lesdits moyens de transmission (12) comportant une liaison sans fil.

3. Dispositif de signalisation selon la revendication 2 dans lequel la liaison entre les première et seconde parties (2,3) est codée pour éviter les erreurs d'appairage.

4. Dispositif de signalisation selon l'une ou l'autre des revendications 1 et 2 dans lequel les moyens de mesure (10) comportent une pince ampérométrique permettant de mesurer l'intensité du courant requis par l'appareil à émission (6).

5. Dispositif de signalisation selon l'une quelconque des revendications précédentes dans lequel les moyens de traitement (11) comportent au moins deux seuils dont un premier seuil bas (S1) est inférieur à la demande minimale en courant lorsque l'appareil à émission (6) est en veille et un second seuil haut (S2) est inférieur à la demande minimale en courant de l'appareil à émission (6) en utilisation.

6. Dispositif de signalisation selon la revendication 5 dans lequel les moyens de traitement (11) permettent un autoapprentissage des seuils bas et haut lors respectivement de la première mise en veille et mise en fonctionnement de l'appareil à émission (6).

7. Dispositif de signalisation selon la revendication 5 dans lequel les moyens de traitement (11) comportent une mémoire comportant des seuils haut et bas fixes préenregistrés en fonction des appareils à émission (6) auquel le dispositif de signalisation (1) correspond.

8. Dispositif de signalisation selon l'une quelconque des revendications précédentes dans lequel les moyens de signalisation (13) sont de type sonore et/ou lumineux et/ou un afficheur de messages.

9. Dispositif de signalisation selon l'une quelconque des revendications précédentes dans lequel la première partie (2) comportent des moyens de coupure de l'alimentation de l'appareil à émission (6) couplés à des moyens de détection de présence dans la zone de champs ouvert de l'appareil à émission (6).

10. Ensemble comportant une unité centrale (15) permettant de recevoir le signal émis par au moins une première partie (2) du dispositif de signalisation (1) selon l'une quelconque des revendications précédentes et permettant de collecter les données de statut relatives à plusieurs dispositifs de signalisation (1).

## Patentansprüche

1. Vorrichtung zur Signalisierung des Status einer Funkübertragungseinrichtung in die Umgebung und insbesondere einer Einrichtung mit einer Röntgenröhre, **dadurch gekennzeichnet, dass** sie Folgendes umfasst:
- einen ersten Teil (2), der mit dem Versorgungskabel (9) der Übertragungseinrichtung verbunden werden kann, und der Messmittel (10) des Strombedarfs der Übertragungseinrichtung, Verarbeitungsmittel (11) zum Bestimmen des Status ausgeschaltet, außer Betrieb, Standby oder in Gebrauch, der Übertragungseinrichtung in Abhängigkeit von der Strommessung und Übertragungsmittel (12) des erfassten Status an einen zweiten Teil (3) aufweist,
- einen zweiten Teil (3), der Signalisierungsmittel (13) des Status der durch den ersten Teil erfassten Übertragungseinrichtung umfasst.

2. Vorrichtung zur Signalisierung nach Anspruch 1, wobei der erste (2) und zweite (3) Teil in separaten Gehäusen (4,5) untergebracht sind, wobei die Übertragungsmittel (12) eine drahtlose Verbindung aufweisen.

3. Vorrichtung zur Signalisierungnach Anspruch 2, wobei die Verbindung zwischen dem ersten und zweiten Teil (2,3) kodiert ist, um Kopplungsfehler zu vermeiden.

4. Vorrichtung zur Signalisierung nach dem einen oder anderen der Ansprüche 1 und 2, wobei die Messmittel (10) eine amperometrische Klemme zum Messen der Intensität des von der Übertragungseinrichtung (6) benötigten Stroms aufweisen.

5. Vorrichtung zur Signalisierung nach einem der vorstehenden Ansprüche, wobei die Verarbeitungsmittel (11) mindestens zwei Schwellenwerte aufweisen, von denen ein erster niedriger Schwellenwert (S1) niedriger ist als der Mindeststrombedarf, wenn sich die Übertragungseinrichtung (6) im Standby-Modus befindet, und ein zweiter hoher Schwellenwert (S2) niedriger ist als der Mindeststrombedarf der Übertragungseinrichtung (6) im in-Gebrauch-Modus.

6. Vorrichtung zur Signalisierung nach Anspruch 5, wobei die Verarbeitungsmittel (11) ein Selbstlernen der niedrigen und hohen Schwellenwerte jeweils während der ersten In-Standby-Schaltung und der Inbetriebsetzung der Übertragungseinrichtung (6) ermöglichen.

7. Vorrichtung zur Signalisierung nach Anspruch 5, wobei die Verarbeitungsmittel (11) einen Speicher mit festen oberen und unteren Schwellenwerten aufweisen, die gemäß den Übertragungseinrichtungen (6), denen die Vorrichtung zur Signalisierung (1) entspricht, vorab aufgezeichnet sind.

8. Vorrichtung zur Signalisierung nach einem der vorstehenden Ansprüche, wobei die Signalisierungsmittel (13) akustischer und/oder optischer Art und/oder eine Nachrichtenanzeige sind.

9. Vorrichtung zur Signalisierungnach einem der vorstehenden Ansprüche, wobei der erste Teil (2) Mittel zum Trennen der Stromversorgung der Übertragungseinrichtung (6) aufweist, die mit Erfassungsmitteln von Anwesenheit im offenen Feldbereich der Übertragungseinrichtung (6) gekoppelt sind.

10. Anordnung mit einer Zentraleinheit (15) zum Empfangen des von mindestens einem ersten Teil (2) der Vorrichtung zur Signalisierung (1) emittierten Signals nach einem der vorstehenden Ansprüche und zum Sammeln von Statusdaten bezüglich mehrerer Vorrichtungen zur Signalisierung (1).

## Claims

1. Device for signalling into the environment the status of a radio transmission apparatus, and in particular of an apparatus provided with an X-ray tube, **characterised in that** it comprises:
- a first part (2), able to be connected at the power cable (9) of said transmission apparatus, and comprising means for measuring (10) the current demand of said transmission apparatus, processing means (11) making it possible to determine the status, switched off, on standby or in use, of said transmission apparatus depending on the current measurement and means for transmitting (12) the detected status to a second part (3),
- a second part (3) comprising means for signalling (13) the status of the transmission apparatus detected by said first part.

2. Device for signalling according to claim 1 wherein the first (2) and the second (3) part are housed in independent cases (4, 5), said means for transmitting (12) comprising a wireless connection.

3. Device for signalling according to claim 2 wherein the connection between the first and second parts (2, 3) is encoded in order to prevent paring errors.

4. Device for signalling according to one or the other of claims 1 and 2 wherein the means for measuring (10) comprise an amperometric clamp making it possible to measure the intensity of the current required by the transmission apparatus (6).

5. Device for signalling according to any preceding claim wherein the processing means (11) comprise at least two thresholds of which a first low threshold (S1) is less than the minimum current demand when the transmission apparatus (6) is on standby and a second high threshold (S2) is less than the minimum current demand of the transmission apparatus (6) in use.

6. Device for signalling according to claim 5 wherein the processing means (11) allow for a self-learning of the low and high thresholds when respectively of the first putting on standby and putting into operation of the transmission apparatus (6).

7. Device for signalling according to claim 5 wherein the processing means (11) comprise a memory comprising pre-recorded fixed high and low thresholds according to the transmission apparatuses (6) to which the signalling device (1) corresponds.

8. Device for signalling according to any preceding claim wherein the means for signalling (13) are of the audible and/or light and/or message display type.

9. Device for signalling according to any preceding claim wherein the first part (2) comprises means for cutting off the power of the transmission apparatus (6) coupled to means for detecting the presence in the zone of open fields of the transmission apparatus (6).

10. Unit comprising a central unit (15) making it possible to receive the signal transmitted by at least one first part (2) of the signalling device (1) according to any preceding claim and making it possible to collect the status data concerning several signalling devices (1).
